# EUROPEAN PATENT APPLICATION

(11) **EP 1 332 729 A1**
(43) Date of publication of application: **06.08.2003**
(21) Application number: 03000383.4
(22) Date of filing: 10.01.2003
(51) Int. Cl.: A61F 2/08

(54) **Device for femoral fixation of the anterior or posterior cruciate neoligament for arthroscopic reconstruction of the cruciate ligament**

(30) Priority: 16.01.2002 IT VI20020006
(71) Applicant: Mikai Manufacturing Co. S.R.L., 35134 Padova (IT)
(72) Inventor: Salvaneschi, Claudio, 16146 Genova (IT); Mussolin, Pietro, 36050 Monteviale (Vicenza) (IT); Miglietta, Carlo, 73100 Lecce (IT)
(74) Representative: Forattini, Amelia

(57) **Abstract**

A device for femoral fixation of the anterior or posterior cruciate neoligament for arthroscopic reconstruction of the cruciate ligament, having a cylindrical body provided with a longitudinal groove and with a slot formed at the end of the cylindrical body, for the passage of a neoligament within the slot. The cylindrical body is adapted to be inserted in the femoral canal.

## Description

The present invention relates to a device for femoral fixation of the anterior or posterior cruciate neoligament for arthroscopic reconstruction of the cruciate ligament.

It is known that in case of rupture or lesion of the anterior or posterior cruciate ligaments the currently employed surgical reconstruction technique entails, among others, the use of two possible replacements of the original ligament: a portion of the patellar tendon or the semitendinosus and gracilis tendon, both taken from the patient on whom the reconstruction is to be performed.

In all cases it is still necessary to form two tunnels, one in the tibia from the outside inward, with access to the articulation, and one in the femur from the inside outward.

In the context of the technique that uses the semitendinosus and gracilis tendon, in which the device according to the present invention is used, various devices are currently available which provide for various alternative femoral fixation techniques.

All these techniques entail the following initial surgical steps:
1. harvesting of the semitendinosus and gracilis tendon that will constitute the future neoligament;
2. creation of the tibial tunnel;
3. creation of the femoral tunnel while the knee is flexed at 90 degrees.

This is followed by alternative surgical steps, depending on the different fixation device that is used.

Conventional devices can be classed into the types described hereafter.

A first type is known as an interference screw.

In this case, the three steps described above are followed by a fourth step for pulling the neoligament into the tunnels, from the tibia to the femur, by way of the suture filament from a previously reinforced end, and by a fifth step for inserting in the femoral tunnel the interference screw, which by interference (hence the name) compresses the neoligament against the wall of the tunnel.

A second conventional device is constituted by a partially threaded narrow transcondylar bar.

In this case, the three previously described steps, which are common to all fixation devices, are followed by a fourth step for passing a metallic wire at right angles to the femoral tunnel by way of an appropriate drilling instrument, producing a second access to the femur. This is then followed by a fifth step for pulling the metallic wire into the transcondylar tunnel. In a sixth step, the metallic wire is taken from the femoral tunnel to the tibial tunnel by way of an appropriate hook. The seventh step consists in pulling the neoligament into the tunnels, from the tibia to the femur, so that it straddles a metallic wire, tensioning the wire through a transcondylar access that is perpendicular to the femoral tunnel. The final steps entail the threading, along the tensioned metallic wire, of the narrow transcondylar bar, which the neoligament straddles, and a step for screwing the threaded end of the bar into the lateral cortex of the femoral condyle.

A third conventional device for femoral fixation of the neoligament is constituted by narrow transcondylar bars.

In this case, the fourth step of the surgical treatment entails pulling the neoligament inside the tunnels, from the tibia to the femur, by way of a suture filament from a previously reinforced end; a fifth step for the passage of a bar at right angles to the femoral tunnel through the neoligament by way of a suitable drilling instrument (therefore producing a second access to the femur); a sixth step for the passage of a second bar at right angles to the femoral tunnel through the neoligament by way of a suitable drilling instrument (therefore producing a third access to the femur).

A fourth conventional femoral fixation device is instead constituted by a transfemoral anchor.

In this case, the fourth step of the surgical treatment requires the produced femoral tunnel to be a through tunnel, i.e., from the articulation outward; this fourth step is followed by a fifth step for inserting the neoligament so that it straddles a slot provided in the device, a sixth step for drawing through the tibial tunnel and the femoral tunnel up to the external vertical plane of the device and of the neoligament, and finally by a seventh step for anchoring on the outer side of the vertical plane the device and therefore the ligament.

All of the steps described above, for all fixation devices, are followed by the fixation of the ligament on the tibia, which is independent of the type of femoral fixation used.

The surgical step for insertion and locking onto the bone ends is followed, for all techniques, by the postoperative step for rehabilitation of the articulation of the patient.

During this period it is essential to activate a biological process during which the neoligament must undergo osteointegration. The neoligament must therefore anchor itself biologically to the bone tissue by which it is surrounded fully or partially, depending on the device that is used. Osteointegration is evaluated by way of appropriate radiological examinations.

All the described techniques, except the one related to the use of the first known fixation device, entail at least one second access to the femur. This increases the risk of surgical failure due to infection, since the risks of penetration of infectious agents increase.

Moreover, the fixation device constituted by an interference screw can cut the neoligament through the turns of the thread of the screw itself. Furthermore, the neoligament is in direct contact with the bone tissue only on the free side, since part of the tunnel is occupied by the screw: this can entail a delay in osteointegration.

In the third conventional fixation device described above, the bars pierce the neoligament, partially cutting the fibers of collagen that are a substantial component for mechanical strength.

Finally, the fourth conventional device entails fixation of the neoligament very far from the articulation, with an increase in the possibility of small movements of the neoligament due to the elasticity of the slot through which the neoligament passes, with a possible increase in osteointegration times.

Moreover, the fourth conventional device, due to the distance of the fixation of the ligament with respect to the articular protrusion, can cause a so-called windshield-wiper effect of oscillation and swinging of the ligament, which can widen the hole of the femoral tunnel at the articulation, causing a consequent looseness of the ligament.

The first, second and fourth conventional fixation devices are generally made of titanium or metal alloy: this entails a risk of delay in osteointegration, because the metal does not undergo osteointegration or resorption; there is also the danger of releasing metal ions, which are a possible cause of local or systemic disorders (metallosis, allergies).

Finally, the metal, in case of radiographic examinations with CAT or MR units, is radioopaque, forming artifacts that do not always allow radiographs to be readable correctly.

The third conventional device and optionally the first device are constituted by synthetic materials of the so-called resorbable type, i.e., they can be metabolized by the human body over a period of time that is not always determinable. This period is not always sufficient for complete osteointegration of the neoligament; moreover, the result of the resorption might be, instead of a bony tissue, a fibrous tissue, which can compromise the possibilities of mechanical strength of the neoligament of the region in which it is fixed.

The aim of the present invention is to provide a device for femoral fixation of the anterior or posterior cruciate neoligament for arthroscopic reconstruction of the cruciate ligament, which allows to reduce surgical times with respect to implantation techniques that use conventional fixation systems.

An object of the present invention is to provide a device for femoral fixation of the anterior or posterior cruciate neoligament, for arthroscopic reconstruction of the cruciate ligament, which allows to eliminate the risks of lesion affecting the neoligament.

A further object of the present invention is to provide a device for femoral fixation of the anterior or posterior cruciate neoligament for arthroscopic reconstruction of the cruciate ligament that allows to obtain adequate fixation of the device by way of the direct interference of the device with the bone tunnel.

A further object of the present invention is to provide a device for femoral fixation of the anterior or posterior cruciate neoligament for arthroscopic reconstruction of the cruciate ligament that allows to leave an adequate contact surface between the bone and the neoligament, so as to rapidly activate the osteointegration process.

A further object of the present invention is to provide a device for femoral fixation of the anterior or posterior cruciate neoligament that is highly reliable, relatively simple to produce and at competitive costs.

This aim and these and other objects that will become better apparent hereinafter are achieved by a device for femoral fixation of the cruciate neoligament for arthroscopic reconstruction of the cruciate ligament, as claimed in the appended claims.

Characteristics and advantages of the invention will become better apparent from the description of a preferred but not exclusive embodiment of the fixation device according to the present invention, illustrated by way of non-limitative example in the accompanying drawings, wherein:

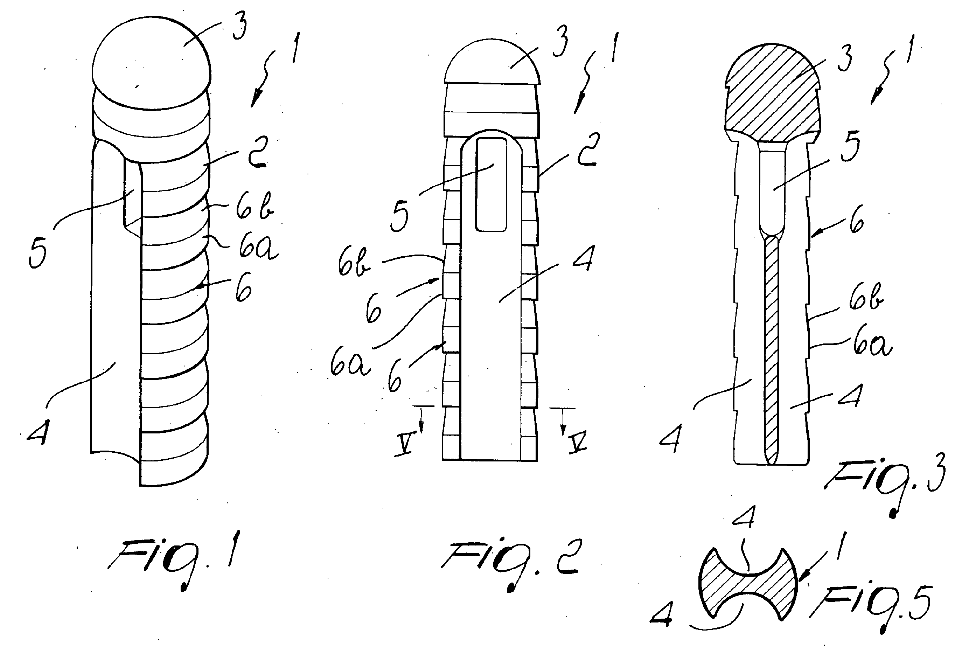

Figure 1 is a perspective view of the fixation device according to the present invention;

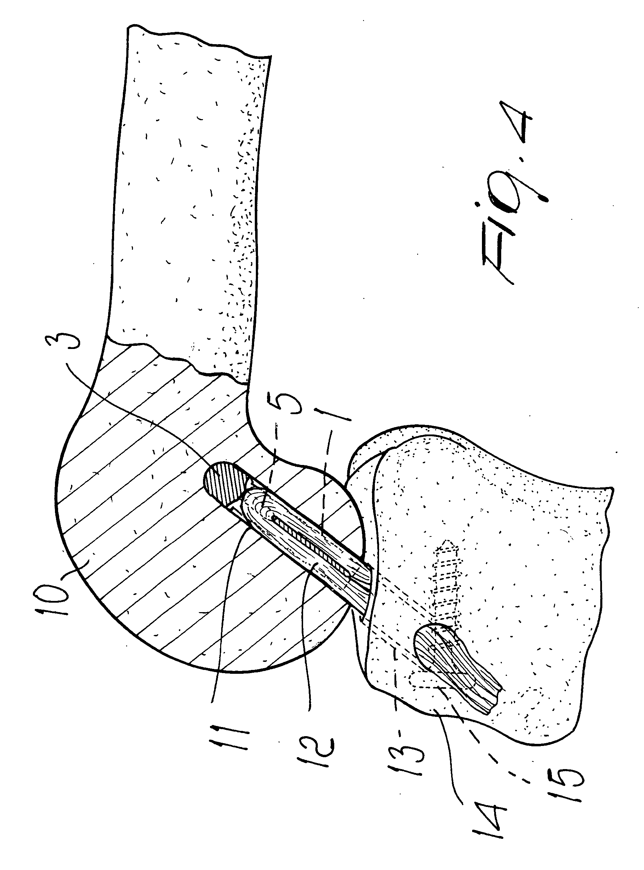

Figure 2 is a front view of the fixation device according to the present invention;
Figure 3 is a sectional view, taken along a transverse plane, of the fixation device according to the present invention;
Figure 4 is a partially sectional view of the use of the fixation device according to the present invention, in the arthroscopic reconstruction of the anterior cruciate ligament;
Figure 5 is a sectional view, taken along a transverse plane, of the device according to the present invention.

With reference to the figures, a femoral fixation device according to the present invention, generally designated by the reference numeral 1, includes a substantially cylindrical body 2 which has a rounded end 3 and two opposite longitudinal semicircular grooves 4, as shown in detail in the sectional plan view of Figure 5.

At the grooves 4, substantially in the region that is directly adjacent to the spherical end portion 3, there is a slot 5.

Conveniently, the outer surface of the cylindrical body 2 is formed by sections 6 constituted by a portion 6a, which is substantially parallel to the diametrical axis of the cylindrical body 2, and by a tapered portion 6b.

The various sections per se, therefore, constitute a sort of screw thread, which however has no sharp parts and allows the anchoring of the fixation device 1 inside the femoral canal, as shown in Figure 4. In this figure, the reference numeral 10 designates the femur of a patient, with the fixation device 1 inserted in the femoral canal 11 produced in order to perform arthroscopy.

The neoligament 12, taken from the body of the same patient, is therefore passed through the slot 5 of the fixation device 1, and is then fixed, by passing within the tibial canal 13 of the tibia 14, inside the tibia by way of a fixation screw 15, according to the conventional technique.

The advantage of the fixation device according to the present invention is therefore that it allows to reduce the time required to install the device inside the femoral canal, since once the femoral canal or tunnel 11 has been formed it is possible to insert the device in the tunnel after passing one end of the neoligament 12 within the slot 5 and resting it inside the mutually opposite grooves 4.

The provision of the mutually opposite grooves 4 allows to eliminate the risks of lesion affecting the neoligament 12.

Moreover, the grooves 4 allow the neoligament 12 to make direct contact with the bone tunnel formed in the femur 10, so as to achieve rapid osteointegration, which is also facilitated by the fact that the contact surface between the bone of the femur and the neoligament 12 is sufficiently large.

The device according to the invention can be provided in different sizes in order to adapt in the best possible manner to the neoligament 12 to be implanted.

The material must have biocompatibility characteristics, but there is substantially no preferred material.

However, the fixation device 1 might be made of animal (or human) bone tissue. This last material would allow to achieve all of the above described advantages and, in addition, a substantial increase in the possibilities of osteointegration of the neoligament 12.

In practice it has been found that the fixation device according to the invention fully achieves the intended aim and objects, since it allows to connect thereto the neoligament without risks of lesion for the ligament and with reduced surgery times.

The fixation device thus conceived is susceptible of numerous modifications and variations, within the scope of the appended claims. All the details may be replaced with other technically equivalent elements.

In practice, the materials employed, so long as they are compatible with a specific use, as well as the contingent shapes and dimensions, may be any according to requirements and to the state of the art.

## Claims

1. A device for femoral fixation of the cruciate neoligament for arthroscopic reconstruction of the cruciate ligament, **characterized in that** it comprises a substantially cylindrical body provided with at least one longitudinal groove and with a slot formed at the end of said cylindrical body, for the passage of a neoligament within said slot, said cylindrical body being adapted to be inserted in the femoral canal.

2. The device according to claim 1, **characterized in that** it comprises two mutually opposite and longitudinal grooves.

3. The device according to claim 1, **characterized in that** said cylindrical body has a spherical end.

4. The device according to one or more of the preceding claims, **characterized in that** said cylindrical body is constituted by a plurality of sections formed by a first portion, which is substantially parallel to the diametrical axis of said cylindrical body, and by a second tapered portion, said sections constituting a means for fixing said fixation device inside said femoral canal.

5. The device according to one or more of the preceding claims, **characterized in that** it is made of biocompatible material.

6. The device according to one or more of the preceding claims, **characterized in that** it is made of bone tissue.

7. The device according to one or more of the preceding claims, **characterized in that** said grooves have a circular arc-like cross-section.
